# FASCICULE DE BREVET EUROPEEN

(11) **EP 3 157 513 B1**
(45) Date de publication et mention de la délivrance du brevet: **15.08.2018**
(21) Numéro de dépôt: 15736546.1
(22) Date de dépôt: 08.06.2015
(51) Int. Cl.: A61K 31/13, A61K 31/21, A61K 33/00, A61P 35/00, A61K 45/06, A61K 9/00

(54) **XÉNON ASSOCIÉ À UN ANTAGONISTE DES RÉCEPTEURS NMDA POUR LUTTER CONTRE UNE PROLIFÉRATION TUMORALE DANS LE SYSTÈME NERVEUX CENTRAL**
XENON IM ZUSAMMENHANG MIT EINEM NMDA-REZEPTOR-ANTAGONISTEN ZUR STEUERUNG DER TUMORPROLIFERATION IN DAS ZENTRALE NERVENSYSTEM
XENON ASSOCIATED WITH AN NMDA RECEPTOR ANTAGONIST FOR CONTROLLING TUMOUR PROLIFERATION IN THE CENTRAL NERVOUS SYSTEM

(30) Priorité: 20.06.2014 FR 1455709
(43) Date de publication de la demande: 26.04.2017
(73) Titulaire: L'AIR LIQUIDE, SOCIETE ANONYME POUR L'ETUDE ET L'EXPLOITATION DES PROCEDES GEORGES CLAUDE, 75007 Paris (FR); L'Institut du Cerveau et de la Moelle Epiniere, 75013 Paris (FR)
(72) Inventeur: MICHEL, Patrick, F-75004 Paris (FR); LAVAUR, Jérémie, F-75007 Paris (FR); HIRSCH, Etienne, F-78000 Versailles (FR); LEMAIRE, Marc, F-75014 Paris (FR)
(74) Mandataire: Pittis, Olivier
(86) Numéro de dépôt international: PCT/FR2015/051515
(87) Numéro de publication internationale: WO 2015/193583

(56) Documents cités:
- EP-A1- 1 552 840
- WO-A1-01/08692
- WO-A2-03/015713
- WO-A2-2005/011711
- TAKANO T ET AL: "Glutamate release promotes growth of malignant gliomas", NATURE MEDICINE, NATURE PUBLISHING GROUP, NEW YORK, NY, US, vol. 7, no. 9, 1 septembre 2001 (2001-09-01), pages 1010-1015, XP002970005, ISSN: 1078-8956, DOI: 10.1038/NM0901-1010
- DINSE A ET AL: "Xenon reduces glutamine-, AMPA-, and Kainate-induced membrane currents in cortical neurones", BRITISH JOURNAL OF ANAESTHESIA, BJM PUBLISHING GROUP, LONDON, GB, vol. 94, no. 4, 1 avril 2005 (2005-04-01), pages 479-485, XP008121311, ISSN: 0007-0912, DOI: 10.1093/BJA/AEI080 [extrait le 2005-02-04]

## Description

L'invention porte sur l'utilisation de xénon gazeux en tant que médicament inhalable utilisé en combinaison avec un antagoniste des récepteurs N-Methyl-D-Aspartate (NMDA) au glutamate, à savoir la mémantine ou la nitromémantine, pour traiter ou ralentir une prolifération tumorale du système nerveux central, en particulier de cellules gliales de type astrocytaire et/ou de leurs précurseurs.

Les récepteurs/canaux NMDA sont des entités moléculaires de la membrane plasmique des cellules neuronales. Ces récepteurs, sont la cible des molécules de glutamate libérées dans l'espace synaptique et extra-synaptique, le glutamate étant un neurotransmetteur excitateur assurant la communication d'une cellule nerveuse à une autre.

Les récepteurs/canaux NMDA sont également présents sur les cellules gliales qui sont des cellules non neuronales du système nerveux central (Lee et al, PloSOne, 2010 ; Dzamba et al, Curr Neuropharmacol, 2013).

La stimulation des récepteurs NMDA sur les cellules gliales de type astrocyte, contribue au contrôle de la libération de glutamate par les terminaisons nerveuses axonales et à la modulation de la plasticité cérébrale (Conti et al, Cereb. Cortex, 1999).

Cette fonction est cependant dérégulée dans les tumeurs des cellules gliales de type astrocytaire et/ou de leurs précurseurs (Takano et al, Nat Med, 2001 ; Rzeski et, Biochem Pharmacol, 2002). En effet, il a été observé qu'un blocage des récepteurs NMDA au glutamate par différents antagonistes de ces récepteurs, notamment la mémantine, réduit sensiblement la prolifération des cellules tumorales gliales de type astrocytaire et/ou de leurs précurseurs, via un mécanisme qui requiert une réduction du flux calcique dans ces cellules (Rzeski et al, Proc. Natl. Acad. Sc. USA, 2001). Le document WO 03/15713 divulgue l'utilisation des antagonistes des récepteurs NMDA, dont la mémantine pour traiter ou ralentir la prolifération tumorale des cellules gliales du système nerveux central. Cependant, l'effet de la mémantine est limité car cette molécule n'est pas dénuée d'effets indésirables, tel que confusion, vertiges, somnolence, céphalées, insomnies, agitation, hallucinations, vomissements, anxiété, ...

Le problème est donc de proposer un traitement, permettant de ralentir ou de minimiser toute prolifération tumorale des cellules du système nerveux central, en particulier de cellules gliales de type astrocytaire et/ou de leurs précurseurs, en limitant les doses de mémantine utilisées et donc les effets indésirables liés à l'utilisation de ce composé, de manière à préserver l'intégrité et les fonctions du tissu nerveux chez un être humain, i.e. un patient humain.

La solution selon la présente invention concerne une combinaison médicamenteuse comprenant du xénon gazeux et au moins un antagoniste des récepteurs NMDA sous forme liquide ou solide pour traiter ou ralentir une prolifération tumorale du système nerveux central, en particulier de cellules gliales, en particulier de cellules gliales de type astrocytaire et/ou de leurs précurseurs, chez un être humain, l'antagoniste des récepteurs NMDA étant la mémantine ou la nitromémantine qui est un composé dérivé de la mémantine.

Dit autrement, la présente invention porte sur un médicament contenant du xénon gazeux pour une utilisation par inhalation en association avec au moins un antagoniste des récepteurs NMDA sous forme liquide ou solide, pour traiter ou ralentir une prolifération tumorale de cellules du système nerveux central, en particulier des cellules gliales, de préférence de type astrocytaire et/ou de leurs précurseurs.

En effet, dans le cadre de la présente invention, il a été mis en évidence que l'association xénon et antagoniste des récepteurs NMDA, à savoir la mémantine ou un composé dérivé de la mémantine, à savoir la nitromémantine, conduit à une action synergique de ces composés et qu'une telle association peut constituer un traitement prometteur pour lutter contre des proliférations tumorales des cellules du système nerveux central, notamment de cellules gliales de type astrocytaire et/ou de leurs précurseurs.

Une telle association repose notamment sur les modes d'action de ces composés.

Ainsi, le xénon possède des propriétés inhibitrices des voies de signalisation glutamatergiques excitatrices via son action antagoniste sur les récepteurs NMDA (Dinse et al, Br J Anaesth, 2005).

Dès lors, l'association xénon/mémantine ou xénon/nitromémantine conduit à une synergie d'action au niveau des récepteurs glutamatergiques, notamment de type NMDA, sans risquer d'augmenter les effets indésirables de la mémantine.

En d'autres termes, le xénon permet, d'augmenter les effets bénéfiques de la mémantine ou de la nitromémantine par un effet synergique mais sans engendrer les effets indésirables de la mémantine ou de la nitromémantine.

Selon le cas, le médicament gazeux selon l'invention peut répondre à l'un ou plusieurs des critères suivants :
- l'antagoniste des récepteurs NMDA est sous forme solide, en particulier sous forme de comprimé ou de gélule.
- l'antagoniste des récepteurs NMDA est la mémantine.
- l'antagoniste des récepteurs NMDA est un composé dérivé de la mémantine, à savoir la nitromémantine.
- l'antagoniste des récepteurs NMDA est la nitromémantine.
- la prolifération tumorale concerne des cellules du système nerveux central.
- la prolifération tumorale concerne des cellules gliales.
- la prolifération tumorale concerne des cellules gliales de type astrocytaire et/ou leurs précurseurs.
- la dose journalière d'antagoniste des récepteurs NMDA administrée au patient est inférieure ou égale à 20 mg/jour.
- le xénon gazeux est administré au patient avant, concomitamment ou après administration de l'antagoniste des récepteurs NMDA, de préférence après administration de l'antagoniste des récepteurs NMDA.
- le xénon est contenu dans un mélange gazeux contenant en outre de l'oxygène, de préférence au moins 21% en volume d'oxygène.
- le mélange gazeux est constitué de xénon et d'oxygène.
- le mélange gazeux est constitué de xénon, d'azote et d'oxygène.
- le patient est un être humain.
- le patient à traiter est un homme ou une femme.
- le xénon est en quantité efficace.
- la quantité de xénon est non anesthésique, i.e. sub-anesthésique.
- elle contient entre 10 et 80% en volume de xénon.
- elle contient entre 15 et 80% en volume de xénon.
- elle contient au moins 20% en volume de xénon.
- elle contient moins de 60% en volume de xénon.
- elle contient de 75% en volume de xénon.
- elle contient entre 20 et 50% en volume de xénon.
- elle contient entre 20 et 40% en volume de xénon.
- le xénon est mélangé avec de l'oxygène juste avant ou au moment de son inhalation par le patient ou se présente sous forme d'un mélange gazeux « prêt à l'emploi » en pré-mélange avec de l'oxygène, et contient éventuellement un autre composé gazeux, par exemple de l'azote.
- le gaz contenant le xénon est administré au patient par inhalation.
- le gaz contenant le xénon est administré au patient, une ou plusieurs fois par jour.
- le gaz contenant le xénon est administré au patient pendant une durée d'inhalation de quelques minutes à quelques heures, typiquement entre 15 minutes et 6 heures, préférentiellement moins de 4 heures.
- la durée, la posologie et la fréquence d'administration sont fonction de l'évolution de l'état neurologique du patient considéré et seront préférentiellement fixées par le médecin ou personnel soignant en fonction de l'état neurologique du patient considéré.
- le xénon est conditionné dans une bouteille de gaz ayant une contenance (équivalent en eau) allant jusqu'à 50 litres, typiquement de l'ordre de 0,5 à 15 litres et/ou à une pression inférieure ou égale à 350 bar absolus, typiquement une pression comprise entre 2 et 300 bar.
- le xénon est conditionné dans une bouteille de gaz équipée d'un robinet ou d'un robinet à détendeur intégré permettant de contrôler le débit et éventuellement la pression du gaz délivré.
- le xénon gazeux est conditionné dans une bouteille de gaz en acier, en aluminium ou en matériaux composites.
- lors du traitement, l'administration du xénon gazeux au patient se fait par inhalation au moyen d'un masque facial ou nasal, de lunettes nasales ou au travers de tout autre système d'administration d'un gaz inhalable.

Selon un autre aspect, l'invention porte également sur une combinaison médicamenteuse telle que définie dans la revendication 1 destinée à être utilisée dans une méthode de traitement thérapeutique pour traiter ou ralentir une prolifération tumorale des cellules du système nerveux central, en particulier des cellules gliales, de préférence des cellules gliales de type astrocytaire et/ou de leurs précurseurs, chez un patient humain, méthode dans laquelle :
i) on identifie un patient humain atteint d'une prolifération tumorale des cellules du système nerveux central, en particulier des cellules gliales de type astrocytaire, et/ou de leurs précurseurs
ii) on administre par inhalation, audit patient, un médicament gazeux contenant du xénon, et
iii) on administre, audit patient, au moins un desdits antagonistes des récepteurs NMDA sous forme liquide ou solide,
de manière à permettre d'inhiber ou de ralentir, la prolifération de type tumorale chez ledit patient.

De préférence, à l'étape i) :
- le patient humain est un enfant, un homme ou une femme.
- l'identification du patient se fait par un médecin ou analogue.
- l'identification de la prolifération se fait par imagerie, examens biologiques, biopsies...
- la prolifération tumorale des cellules du système nerveux central s'observe en particulier au niveau des cellules gliales de type astrocytaire et/ou de leurs précurseurs.
- la prolifération tumorale des cellules du système nerveux central, résulte d'une entrée excessive d'ions Ca²⁺ dans des cellules de type astrocytaire.

De préférence, à l'étape ii) :
- on administre, audit patient, au moins un desdits antagonistes des récepteurs NMDA sous forme solide.
- on administre au moins un desdits antagonistes des récepteurs NMDA, de préférence par voie entérale, i.e. la voie orale.
- on administre, audit patient, au moins un desdits antagonistes des récepteurs NMDA sous forme de comprimé ou de gélule.
- on administre au patient de la mémantine ou de la nitromémantine en tant qu'antagoniste des récepteurs NMDA.
- on administre au patient une dose journalière d'antagoniste des récepteurs NMDA inférieure ou égale à 20 mg/jour.
- on administre, audit patient, au moins un desdits antagonistes des récepteurs NMDA avant, concomitamment, ou après inhalation de xénon par le patient.

De préférence, à l'étape iii) :
- la durée, la posologie et la fréquence d'administration du xénon sont choisies et/ou fixées en fonction de l'évolution de l'état neurologique du patient considéré.
- on administre une quantité efficace de xénon.
- on administre une quantité non anesthésique de xénon.
- on administre de 10 à 75% en volume de xénon, de préférence entre 20 et 50% en volume de xénon.
- on mélange le xénon avec de l'oxygène avant ou au moment de son inhalation par le patient, de préférence avec au moins 21% en volume d'oxygène.
- on administre un mélange gazeux prêt à l'emploi constitué de xénon et d'oxygène (mélange binaire) ou de xénon, d'oxygène et d'azote (mélange ternaire).
- on administre le xénon gazeux une ou plusieurs fois par jour au patient.
- on administre le xénon gazeux au patient pendant une durée d'inhalation de quelques minutes à quelques heures, typiquement entre 15 minutes et 6 heures, préférentiellement moins de 4 heures.
- on administre le xénon gazeux au moyen d'un masque facial ou nasal, de lunettes nasales ou au travers de tout autre système ou dispositif d'administration de gaz à un patient.

L'invention va maintenant être mieux comprise grâce aux Exemples suivants et en référence aux Figures annexées, donnés à titre illustratif, parmi lesquelles :
- la Figure 1 illustre l'efficacité d'une combinaison du xénon et d'un antagoniste des récepteurs NMDA dans un modèle cellulaire mimant une prolifération cellulaire de cellules gliales de type astrocytaire, et/ou de leurs précurseurs
- la Figure 2 illustre l'efficacité d'une combinaison du xénon et d'un antagoniste des récepteurs NMDA dans un modèle cellulaire de culture mixte neurone-glie.

### Exemple 1

Afin de démontrer l'efficacité de la combinaison du xénon et d'un antagoniste des récepteurs NMDA selon la présente invention, on a mis en place un modèle cellulaire de cultures enrichies en cellules de type astrocytaire, dans lequel la prolifération est amplifiée et entretenue par la présence de mitogènes, contenus notamment dans le sérum de veau foetal (SVF) qui sert à supplémenter le milieu de culture.

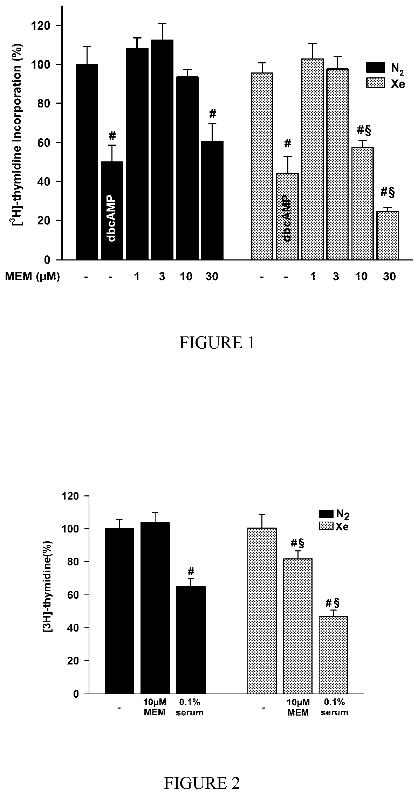

La technique mise en place est décrite ci-après et les résultats obtenus sont consignés dans le Tableau 1 et illustrés en Figure 1.

Ces résultats montrent les effets anti-prolifératifs synergiques du xénon et de la mémantine dans un modèle cellulaire mimant une prolifération de cellules gliales de type astrocytaire et/ou de leurs précurseurs

### Protocole d'obtention des cultures primaires d'astrocytes

Des cultures sont préparées à partir de cerveau total d'embryons, prélevés sur des rates gestantes Wistar, au jour 15,5 de gestation.

Le procédé de production des cultures enrichies en astrocytes (cellules immuno-positives pour la « Glial fibrillary acidic protein » ou GFAP) comprend l'obtention d'une suspension cellulaire homogène de tout le cerveau antérieur embryonnaire par dissociation mécanique, c'est-à-dire non enzymatique, en utilisant du milieu L15 de Leibovitz (Sigma Aldrich).

Un aliquote de suspension cellulaire correspondant au tissu cérébral provenant de 5 embryons, est ajouté à chaque flasque (Costar) de 25cm² contenant 5 ml d'un milieu nourricier qui va permettre de stimuler la prolifération des astrocytes et de leurs précurseurs (Sergent-Tanguy et al, J Neurosci Res, 2006), tout en éliminant les cellules neuronales ; les neurones n'adhèrent pas sur le fond des flasques en absence de coating, à la différence des astrocytes. Le milieu utilisé est du Minimum Essential Medium (MEM), contenant 10% de sérum de veau foetal (SVF, Biowest) et 10 % de sérum de cheval (SC, Sigma Aldrich), 1g/l de glucose, 2 mM de L-glutamine, 1mM de pyruvate de sodium, des acides aminés non-essentiels, un cocktail pénicilline/streptomycine. Lorsque la confluence est atteinte (c'est-à-dire après 7-10 jours de culture), les cellules adhérentes (>90% d'astrocytes) sont placées dans du PBS, puis détachées en tapotant les flasques. Les cellules récupérées sont centrifugées et triturées mécaniquement avant mise en culture. Les astrocytes sont alors cultivés dans des boites multi-puits de 48 puits (Nunc). Environ 30000 cellules sont appliquées par puits de culture. Les cellules sont mises en présence du même milieu que précédemment mais avec une supplémentation en sérums, réduite à 2 %.

Aucun changement de milieu de culture n'est effectué pendant toute la période de la culture.

### Traitements pharmacologiques des cultures

La prolifération est déclenchée et entretenue par la présence de SVF dans le milieu de culture.

Le bloqueur des récepteurs NMDA, la mémantine (RD Systems) est ajoutée aux cultures juste avant l'exposition au xénon. Le dibutyryl cyclic AMP (dbcAMP), une forme lipophile d'AMPc utilisée comme molécule anti-proliférative de référence (Mourlevat et al, Mol Pharmacol, 2003), est ajouté au même moment que la mémantine.

### Maintenance des cultures sous atmosphère gazeuse contrôlée

Une fois les traitements pharmacologiques effectués, les boîtes multi-puits contenant les cellules en culture et la boite servant à l'humidification du compartiment interne de la chambre, sont placées sur une embase métallique qui reçoit la chambre d'incubation en Plexiglas. Les deux pièces (embase et chambre en Plexiglas) sont réunies par vissage, de manière jointive.

On injecte ensuite un mélange gazeux d'intérêt comprenant (% en volume) : 20 % d'O₂, 5 % de CO₂ et 75 % du gaz testé, dans la chambre d'incubation, avec valves d'entrée et de sortie ouvertes, tout en contrôlant le débit de sortie grâce à un débitmètre. Les gaz testés sont de l'azote et du xénon.

Le débit de sortie de référence, fixé pour l'air à 10 litres/mn, est corrigé en fonction de la densité du mélange utilisé.

Lorsque la mesure du CO₂, atteint 5 % en sortie, on stoppe l'injection du mélange gazeux et la chambre est rendue totalement étanche en fermant les valves d'entrée et de sortie.

La chambre d'exposition est alors placée dans une enceinte à 37°C pendant toute la durée du protocole expérimental.

### Mesure de la prolifération cellulaire par accumulation de thymidine tritiée

Après rupture de l'étanchéité par ouverture des valves d'entrée et de sortie et dévissage de la chambre de son embase, les cultures sont mises en présence de PBS contenant 5 mM de glucose et incubées pendant 2 hrs avec de la thymidine tritiée (Perkin, 70-90Ci/mmol ; 0.5 µCi/puit) pour analyse de la prolifération cellulaire selon une méthode préalablement décrite par Rousseau et collègues (Mol Pharmacol, 2013).

Les résultats obtenus dans le modèle de prolifération cellulaire en présence de SVF, révèlent des effets inhibiteurs synergiques entre le xénon et la mémantine qui sont résumés dans le Tableau 1 suivant et représentés sur la Figure 1.

**Tableau 1: Résumé des principaux résultats de l'étude in vitro sur des cultures enrichies en astrocytes**

| Cultures enrichies en astrocytes à J3 | | |
|---|---|---|
| Traitements J 0 - J 3 (3 jours) | Mélange gazeux (20% O₂ + 5% CO₂ + 75 % gaz testé) ; % en volume | Réduction de la prolifération |
| Groupe témoin I | N₂ | - |
| Groupe témoin II | Xe | - |
| dbcAMP (1 mM) | N₂ | ++ |
| dbcAMP (1 mM) | Xe | ++ |
| Mémantine (1 µM) | N₂ | - |
| Mémantine (1 µM) | Xe | - |
| Mémantine (3 µM) | N₂ | - |
| Mémantine (3 µM) | Xe | - |
| Mémantine (10 µM) | N₂ | - |
| Mémantine (10 µM) | Xe | ++ |
| Mémantine (30 µM) | N₂ | ++ |
| Mémantine (30 µM) | Xe | +++ |

Dans le tableau précédent, une réponse favorable, synonyme d'une inhibition de la prolifération des cellules gliales de type astrocytaire en présence des traitements d'intérêt, est désignée par un signe « + », « ++ », « +++ », ou «++++ » (++++ = absence de prolifération). A l'inverse, une absence de réponse est représentée par un signe « - », synonyme d'une non efficacité vis-à-vis de la prolifération cellulaire.

La mémantine agit en bloquant les récepteurs NMDA et le xénon en bloquant ces mêmes récepteurs mais probablement via un mécanisme distinct, impliquant le blocage du site de fixation de la glycine, un acide aminé qui agit comme coactivateur du récepteur.

Le SVF, induit et entretient le processus de prolifération cellulaire du fait de son fort contenu en facteurs mitogènes.

Au vu des résultats illustrés en Figure 1, on constate que la combinaison du xénon et d'un antagoniste des récepteurs NMDA, à savoir ici la mémantine, conduit à un effet anti-prolifératif synergique supérieur à celui produit par chaque traitement, individuellement. En fait, une réelle synergie d'action de l'association xénon/mémantine se met en place vis-à-vis du mécanisme de prolifération.

Tout d'abord, ces essais ont montré que la prolifération en présence de mitogènes sériques est soutenue sous atmosphère contenant 75 vol.% d'azote. Cette prolifération peut être significativement réduite par un analogue lipophile de l'AMPc, le dibutryl-cAMP.

Lorsque que le xénon remplace l'azote, on n'observe pas d'effet anti-prolifératif significatif.

La mémantine sous atmosphère enrichie en azote n'a pas non plus d'effet anti-prolifératif jusqu'à 10µM. Cependant, un traitement avec 10 µM de mémantine dans une atmosphère enrichie en xénon, conduit à un effet anti-prolifératif significatif (-42,5%).

La mémantine à 30 µM exerce un effet anti-prolifératif à elle seule (-39,3%) dans une atmosphère enrichie en azote et cet effet est fortement potentialisé sous une atmosphère enrichie en xénon (-75,2%).

Les résultats schématisés sur la Figure 1 illustrent les effets protecteurs synergiques du xénon et de la mémantine dans un modèle cellulaire mimant une prolifération de cellules gliales de type astrocytaire et/ou de leurs précurseurs.

Ces résultats ont été obtenus sur des cultures enrichies en cellules gliales de type astrocytaire, obtenues à partir de cerveau de rat. Ces cultures ont été maintenues dès leur mise en culture et jusqu'au J3 *in vitro,* dans une atmosphère contenant 75 % d'azote (N₂ 75) ou 75 % de xénon (Xe 75), en présence ou non de mémantine (MEM), testée à 1, 3, 10 ou 30 µM ou en présence d'un analogue soluble de l'AMPc, le dibutyryl-cAMP, utilisé comme molécule anti-proliférative de référence.

A la fin des traitements, les cultures sont récupérées pour une mesure de prolifération cellulaire, en utilisant comme index d'évaluation l'incorporation de thymidine tritiée.

Les résultats sont exprimés en % (± SEM, n=6) des valeurs moyennes des cultures maintenues sous 75 % d'azote (condition témoin).

Ainsi, l'étude statistique faite par analyse de variance à un facteur ou méthode One-way ANOVA (ANalysis Of VAriance), suivie d'un test de Student-Newman-Keuls Method) pour des comparaisons multiples par paires (n= 6 pour chaque point expérimental) démontre que :
- dans une atmosphère à 75 % d'azote, la prolifération des astrocytes est réduite en présence de 30 µM de mémantine ou de 1 mM de dbcAMP (# p<0.05, vs cultures témoins maintenues sous 75 % d'azote).
- dans une atmosphère à 75 % de xénon, la prolifération des astrocytes est réduite dès 10 µM de mémantine et avec 1 mM de dbcAMP (# p<0.05, vs cultures témoins maintenues sous 75 % d'azote).
- le potentiel anti-prolifératif de la mémantine est soit révélé, soit augmenté sous atmosphère enrichie en xénon (§ p<0.05, vs cultures exposées aux mêmes concentrations de mémantine sous 75 % d'azote).

Le xénon conduit donc, lorsqu'il est associé à de la mémantine ou à de la nitromémantine agissant en tant qu'antagoniste du récepteur NMDA, à un effet synergique dans le traitement ou le ralentissement des proliférations tumorales du système nerveux central de cellules gliales de type astrocytaire et/ou de leurs précurseurs

### Exemple 2

Afin de confirmer la réalité de l'effet synergique de la combinaison xénon et antagoniste des récepteurs NMDA selon la présente invention, on a mis en place un autre modèle cellulaire de culture mixte neurone-glie, dans lequel la prolifération des cellules gliales de type astrocytaire et/ou de leurs précurseurs s'observe en présence de cellules neuronales (Rousseau et al, Mol Pharmacol, 2013).

La technique mise en oeuvre est décrite ci-dessous et les résultats obtenus sont résumés dans le Tableau 2 et illustrés en Figure 2 qui montre les effets inhibiteurs synergiques du xénon et de la mémantine dans un modèle cellulaire mimant une prolifération de cellules gliales de type astrocytaire et/ou de leurs précurseurs en présence de cellules neuronales.

### Protocole d'obtention des cultures mixtes neurone-glie

Des cultures sont préparées à partir de mésencéphale d'embryons de rats, prélevés sur des rates Wistar, au jour 15,5 de gestation.

La mise en oeuvre des cultures de mésencéphale comprend l'obtention d'une suspension cellulaire homogène par dissociation mécanique, c'est-à-dire non enzymatique, du tissu embryonnaire, en utilisant du milieu L15 de Leibovitz (Sigma Aldrich).

Des aliquotes de cette suspension sont ajoutés à des plaques Nunc multi-puits de 48 puits, qui ont été préalablement revêtues d'une fine couche de polyéthylènimine (1 mg/ml, tampon borate pH 8,3) pour permettre l'adhésion des cellules neuronales (cf. Toulorge et coll, Faseb J, 2011).

La densité d'ensemencement est comprise entre environ 80000 et 100000 cellules/ puits de culture.

Les cultures de mésencéphale sont maintenues dans du milieu de culture MEM, contenant 1g/l de glucose, 2 mM de L-glutamine, 1mM de pyruvate de sodium, des acides aminés non-essentiels et un cocktail pénicilline/streptomycine. La première semaine de culture, ce milieu contient aussi 10% de sérum de veau foetal (SVF, Biowest) et 10 % de sérum de cheval (SC, Sigma Aldrich), mais à partir de la seconde semaine les concentrations des deux sérums sont abaissées (Gao et al, J Neurosci, 2002).

Jusqu'au moment où l'on évalue les effets des gaz d'intérêt, les cultures sont placées dans une enceinte conventionnelle thermostatée à 37°C, dans laquelle le CO₂ est maintenu à 5% en volume et où l'atmosphère est saturée en eau.

Le processus de prolifération gliale intervient dès la mise en culture. Il est analysé au cours de la deuxième semaine in vitro lorsque la supplémentation en sérums est réduite à 2% ou dans certains cas à 0,1%.

### Traitements pharmacologiques des cultures

Après réduction des concentrations en SVF et en SC à J7, le bloqueur des récepteurs NMDA, la mémantine est ajoutée aux cultures avant la mise sous atmosphère gazeuse contrôlée.

### Maintenance des cultures sous atmosphère gazeuse contrôlée

Une fois les traitements pharmacologiques effectués, les boîtes multi-puits contenant les cellules en culture et la boite servant à l'humidification du compartiment interne de la chambre, sont placées sur une embase métallique qui reçoit la chambre d'incubation en Plexiglas. Les deux pièces (embase et chambre en Plexiglas) sont réunies par vissage, de manière jointive.

On injecte ensuite un mélange gazeux d'intérêt comprenant (% en volume) : 20 % d'O₂, 5 % de CO₂ et 75 % de gaz testé, dans la chambre d'incubation, avec valves d'entrée et de sortie ouvertes, tout en contrôlant le débit de sortie grâce à un débitmètre. Les gaz testés sont de l'azote et du xénon.

Le débit de sortie de référence, fixé pour l'air à 10 litres/mn, est corrigé en fonction de la densité du mélange utilisé.

Lorsque la mesure du CO₂, atteint 5 % en sortie, on stoppe l'injection du mélange gazeux et la chambre est rendue totalement étanche en fermant les valves d'entrée et de sortie.

La chambre d'exposition est alors placée dans une enceinte à 37°C pendant les 7 jours du protocole expérimental.

### Mesure de la prolifération cellulaire par incorporation de thymidine

A J14, après rupture de l'étanchéité par ouverture des valves d'entrée et de sortie et dévissage de la chambre d'incubation de son embase, les cultures sont soumises au test de prolifération à la thymidine tritiée selon le protocole décrit dans l'exemple 1, en se basant sur la technique décrite par Rousseau et collègues (Mol. Pharmacol., 2013).

Les résultats obtenus dans le modèle de prolifération des cellules astrocytaires, révèlent des effets inhibiteurs synergiques entre le xénon et la mémantine qui sont résumés dans le tableau 2 suivant et représentés sur la Figure 2.

On note aussi que le fait de réduire les concentrations des deux sérums à 0,1 %, réduit fortement la prolifération de type astrocytaire, ce qui démontre le rôle clé des mitogènes sériques dans le processus prolifératif.

**Tableau 2 : Résumé des principaux résultats de l'étude in vitro dans le modèle de cultures mixtes neurone-glie**

| Cultures mixtes neurone-glie J14 | | |
|---|---|---|
| Traitements J 7 - J 14 (7 jours) | Mélange gazeux (20% O₂ + 5% CO₂ + 75 % gaz testé) ; % en volume | Inhibition de la prolifération |
| Groupe témoin, sérums à 2% | N₂ | - |
| Groupe Xe, sérums à 2% | Xe | - |
| Groupe mémantine (10 µM), sérums à 2% | N₂ | - |
| Groupe Xe + mémantine (10 µM), sérums à 2% | Xe | + |
| Groupe, sérums à 0,1 % | N₂ | ++ |
| Groupe Xe, sérums à 0,1 % | Xe | +++ |

Au vu des résultats représentés sur la Figure 2, on constate que la combinaison du xénon et d'un antagoniste des récepteurs NMDA, à savoir ici la mémantine, conduit à un effet inhibiteur synergique vis-à-vis de la prolifération astrocytaire. Le traitement des cultures avec de la mémantine appliquée sous azote ou la maintenance des cultures dans une atmosphère enrichie en xénon n'ont pas d'impact sur la prolifération. L'abaissement des concentrations des sérums du milieu de culture conduit aussi à une réduction de la prolifération de type astrocytaire, démontrant ainsi que la combinaison mémantine/xénon est active contre un mécanisme activé par des mitogènes sériques.

En fait, une réelle synergie d'action de l'association xénon/mémantine se met en place.

Les résultats décrits sur la Figure 2 démontrent les effets inhibiteurs synergiques du xénon et de la mémantine dans le modèle cellulaire de cultures mixtes neurone-glie mimant une prolifération de cellules gliales de type astrocytaire et/ou de leurs précurseurs.

Ces résultats ont été obtenus sur des cultures de mésencéphale de rat qui ont été placées à compter du jour 7 de culture dans un milieu supplémenté par 2% de SVF et de SC sous atmosphère contenant 75 % d'azote (N₂ 75) ou 75 % de xénon (Xe 75), en présence ou non de mémantine (MEM), testée à 10 µM. Certaines expériences ont aussi été réalisées en réduisant les concentrations des 2 types de sérum à 0,1%,, en maintenant les cellules sous atmosphère enrichie en azote ou en xénon.

Les cultures sont ensuite récupérées pour une mesure de la prolifération cellulaire en utilisant de la thymidine tritiée.

Les résultats sont exprimés en % (± SEM) des cultures à J14 maintenues sous azote (condition témoin).

Ainsi, l'étude statistique faite par une ANOVA (ANalysis Of VAriance), suivie d'un test de Fisher (n= 13-15 pour chaque point expérimental) démontre que :
- dans une atmosphère à 75 % d'azote, la prolifération des astrocytes est diminuée seulement si les concentrations en sérums sont abaissées de 2% à 0,1 % (# p<0.05, vs cultures témoins maintenues sous 75 % d'azote).
- dans une atmosphère à 75 % de xénon, la prolifération des astrocytes est diminuée si les concentrations en sérums sont réduites à 0,1 % ou si les cultures sont traitées avec de la mémantine (# p<0.05, vs cultures témoins maintenues sous 75 % d'azote).
- le xénon révèle les effets antiprolifératifs d'un traitement à la mémantine ou améliore ceux produits par abaissement des concentrations en sérums (§ p<0.05, vs cultures correspondantes exposées aux mêmes traitements sous 75 % d'azote).

Le xénon conduit donc, lorsqu'il est associé à un antagoniste des récepteurs NMDA, telle la mémantine ou la nitromémantine agissant en tant qu'antagoniste des récepteurs NMDA, à un effet synergique dans le traitement ou le ralentissement des proliférations tumorales du système nerveux central de cellules gliales de type astrocytaire et/ou de leurs précurseurs.

En d'autres termes, selon l'invention, du xénon gazeux peut être utilisé pour fabriquer un médicament inhalable permettant de traiter ou de ralentir, lorsqu'il est utilisé en association avec au moins un antagoniste des récepteurs NMDA sous forme liquide ou solide, une prolifération tumorale de cellules dans le système nerveux central chez un être humain.

## Revendications

1. Combinaison médicamenteuse comprenant du xénon gazeux et au moins un antagoniste des récepteurs NMDA sous forme liquide ou solide pour son utilisation pour traiter ou ralentir une prolifération tumorale de cellules dans le système nerveux central chez un être humain, l'antagoniste des récepteurs NMDA étant la mémantine ou la nitromémantine.

2. Combinaison médicamenteuse pour son utilisation selon la revendication précédente, **caractérisée en ce qu'**elle contient entre 10 et 80% en volume de xénon.

3. Combinaison médicamenteuse pour son utilisation selon l'une des revendications précédentes, **caractérisée en ce qu'**elle contient un antagoniste des récepteurs NMDA sous forme solide, en particulier sous forme de comprimé ou de gélule.

4. Combinaison médicamenteuse pour son utilisation selon l'une des revendications précédentes, **caractérisée en ce que** les cellules sont des cellules gliales.

5. Combinaison médicamenteuse pour son utilisation selon l'une des revendications précédentes, **caractérisée en ce que** les cellules gliales sont de type astrocytaire et/ou de leurs précurseurs.

6. Combinaison médicamenteuse pour son utilisation selon l'une des revendications précédentes, **caractérisée en ce qu'**elle contient en outre de l'oxygène.

7. Combinaison médicamenteuse pour son utilisation selon l'une des revendications précédentes, **caractérisée en ce qu'**elle contient en outre au moins 21% en volume d'oxygène.

8. Combinaison médicamenteuse pour son utilisation selon l'une des revendications précédentes, **caractérisée en ce que** le xénon est contenu dans un mélange constitué de xénon et d'oxygène ou un mélange constitué de xénon, d'oxygène et d'azote.

9. Combinaison médicamenteuse pour son utilisation selon l'une des revendications précédentes, **caractérisée en ce qu'**elle contient au moins 20% en volume de xénon.

10. Combinaison médicamenteuse pour son utilisation selon l'une des revendications précédentes, **caractérisée en ce qu'**elle contient moins de 75% en volume de xénon, de préférence elle contient moins de 60% en volume de xénon.

## Patentansprüche

1. Arzneimittelkombination, umfassend gasförmiges Xenon und mindestens einen Antagonisten von NMDA-Rezeptoren in flüssiger oder fester Form zur Verwendung zur Behandlung oder Verlangsamung eines Tumorwachstums von Zellen im zentralen Nervensystem bei einem Menschen, wobei es sich beim Antagonisten von NMDA-Rezeptoren um Memantin und Nitromemantin handelt.

2. Arzneimittelkombination zur Verwendung nach dem vorstehenden Anspruch,
**dadurch gekennzeichnet, dass** sie zwischen 10 und 80 Vol.-% Xenon enthält.

3. Arzneimittelkombination zur Verwendung nach einem der vorstehenden Ansprüche,
**dadurch gekennzeichnet, dass** sie einen Antagonisten von NMDA-Rezeptoren in fester Form, insbesondere in Form einer Tablette oder Kapsel, enthält.

4. Arzneimittelkombination zur Verwendung nach einem der vorstehenden Ansprüche,
**dadurch gekennzeichnet, dass** es sich bei den Zellen um Gliazellen handelt.

5. Arzneimittelkombination zur Verwendung nach einem der vorstehenden Ansprüche,
**dadurch gekennzeichnet, dass** die Gliazellen vom Astrozytentyp und/oder deren Vorläufer sind.

6. Arzneimittelkombination zur Verwendung nach einem der vorstehenden Ansprüche,
**dadurch gekennzeichnet, dass** sie darüber hinaus Sauerstoff enthält.

7. Arzneimittelkombination zur Verwendung nach einem der vorstehenden Ansprüche,
**dadurch gekennzeichnet, dass** sie darüber hinaus mindestens 21 Vol-% Sauerstoff enthält.

8. Arzneimittelkombination zur Verwendung nach einem der vorstehenden Ansprüche,
**dadurch gekennzeichnet, dass** das Xenon in einem aus Xenon und Sauerstoff bestehenden Gemisch oder einem aus Xenon, Sauerstoff und Stickstoff bestehenden Gemisch enthalten ist.

9. Arzneimittelkombination zur Verwendung nach einem der vorstehenden Ansprüche,
**dadurch gekennzeichnet, dass** sie mindestens 20 Vol.-% Xenon enthält.

10. Arzneimittelkombination zur Verwendung nach einem der vorstehenden Ansprüche,
**dadurch gekennzeichnet, dass** sie weniger als 75 Vol-% Xenon, vorzugsweise weniger als 60 Vol-% Xenon enthält.

## Claims

1. Drug combination including gaseous xenon and at least one NMDA receptor antagonist in liquid or solid form in order to treat or slow tumour proliferation of cells in the central nervous system in a human being, with the NMDA receptor antagonist being memantine or nitromemantine.

2. Drug combination for its use as claimed in the preceding claim, **characterized in that** it contains between 10 and 80% by volume of xenon.

3. Drug combination for its use according to one of the preceding claims, **characterized in that** it contains an NMDA receptor antagonist in solid form, in particular in tablet or capsule form.

4. Drug combination for its use according to one of the preceding claims, **characterized in that** the cells are glial cells.

5. Drug combination for its use according to one of the preceding claims, **characterized in that** the glial cells are of the astrocyte type and/or the precursors thereof.

6. Drug combination for its use according to one of the preceding claims, **characterized in that** it furthermore contains oxygen.

7. Drug combination for its use according to one of the preceding claims, **characterized in that** it furthermore contains at least 21 % by volume of oxygen.

8. Drug combination for its use according to one of the preceding claims, **characterized in that** the xenon is contained in a mixture comprised of xenon and of oxygen or a mixture comprised of xenon, oxygen and nitrogen.

9. Drug combination for its use according to one of the preceding claims, **characterized in that** it contains at least 20% by volume of xenon.

10. Drug combination for its use according to one of the preceding claims, **characterized in that** it contains less than 75% by volume of xenon, preferably it contains less than 60% by volume of xenon.
